# EUROPEAN PATENT APPLICATION

(11) **EP 1 878 783 A1**
(43) Date of publication of application: **16.01.2008**
(21) Application number: 06117217.7
(22) Date of filing: 14.07.2006
(51) Int. Cl.: C10L 5/40, C10L 9/00, C10G 1/00, C10L 9/02, C10L 9/08, C10L 9/10, C01B 31/08, D01F 9/127

(54) **Modified biomass comprising synthetically grown carbon fibers**

(71) Applicant: BIOeCON International Holding N.V., Curaçao (AN)
(72) Inventor: O'Connor, Paul, 3871 KM, Hoevelaken (NL)
(74) Representative: Rasser, Jacobus Cornelis

(57) **Abstract**

Biomass particles are modified by associating the particles with carbon fibers. The carbon fibers may be coated onto the biomass particles, or may be embedded within the biomass particles.

As a result of the association with carbon fibers the particles are more susceptible to conversion to bioliquid.

## Description

### BACKGROUND OF THE INVENTION

### 1. Field of the Invention

The present invention relates to a process for pretreating a solid biomass material. The pretreatment results in an activated biomass material that is susceptible to conversion to a liquid fuel under mild conditions.

### 2. Description of the Related Art

As the supplies of readily accessible crude oil are dwindling, there is an increasing need for liquid fuels from other sources. Certain carbon-based energy carrier materials are abundantly available. Examples include biomass, in particular biomass of photosynthetic origin, generally comprising cellulose and/or lignocellulose. Processes have been developed to convert these energy carrier materials to liquid fuels. Examples of such processes include pyrolysis and hydrothermal conversion. However, these processes require relatively severe conditions, which require expensive equipment and a high-energy input.

There is, therefore, a need for developing solid materials that are sensitized ('activated'), so as to be more susceptible to conversion to a liquid fuel under relatively mild conditions.

### BRIEF SUMMARY OF THE INVENTION

The present invention relates to a particulate solid biomass material having associated therewith fibers of carbon. The carbon fibers may be coated onto the biomass particles, embedded within the biomass particles, or both. The carbon fibers preferably are nanofibers.

Preferred biomass materials are those of photosynthetic origin, in particular biomass materials comprising cellulose and/or lignocellulose.

The carbon fiber associated solid biomass materials may be prepared by depositing a suitable catalytic material onto particles of the solid biomass material, and contacting the resulting particles with a suitable carbon source.

The solid biomass associated with carbon fibers is more susceptible to conversion to a bioliquid by hydrothermal conversion, enzymatic conversion, mild thermal conversion or catalytic conversion.

### DESCRIPTION OF ILLUSTRATIVE EMBODIMENTS

The following is a description of certain embodiments of the invention, given by way of example only.

The present invention relates to a particulate solid biomass material having associated therewith fibers of carbon. The carbon fibers are preferably nanofibers. The carbon fibers may be coated onto the biomass particles, embedded within the biomass particles, or a combination thereof.

Preferred biomass materials are those of photosynthetic origin, specifically materials comprising cellulose and/or lignocellulose. Examples include aquatic plants such as algae; forestry waste such as wood chips and saw dust; agricultural plant waste such as bagasse, straw, corn cobs, and the like; so-called energy crops such as switch grass; and food crops such as corn and grains.

As a result of the association with carbon fibers the solid biomass material is more susceptible to conversion to, for example, bioliquid. For example, these materials may be converted to bioliquids in a hydrothermal conversion process at temperatures of less than 300 °C, preferably less than 240 °C, or even less than 200 °C. The ability to operate at lower temperatures entails significant cost savings, because hydrothermal conversion is generally carried out at autogenous pressures. The saturated steam pressure at 300°C is 85 bar; at 240 °C it is 33 bar; and at 200 °C it is 15 bar. Accordingly, lower conversion temperatures are associated with significant cost savings as the equipment cost is much lower for reactors designed for the much lower pressures.

Another aspect of the present invention is a process for preparing a solid biomass materials having associated therewith fibers of carbon, said process comprising the steps of:
a) providing a solid biomass material in particulate form;
b) depositing onto said particulate biomass material particles of a catalytic material capable of catalyzing the formation of carbon fibers, to form an activated biomass;
c) contacting the activated biomass with a suitable carbon source.

The biomass particles preferably have a particle size of less than 5 mm, more preferably in the range of 0.1 to 3 mm. The particles are prepared from larger pieces of solid biomass, such as corn husks, straw, wood chips, and the like, by known techniques such as grinding, milling, and the like. In a particularly preferred process the biomass is first comminuted to a particle size of around 5 mm by grinding or milling, and subsequently further reduced in size by abrasion with harder particles, such as sand, in a fluidized bed, an ebullient bed, a spouted bed, or pneumatic conveyance.

Deposition of a catalytic material onto the biomass particles may be done by any suitable method known in the catalysis art. Examples of suitable methods include impregnation followed by drying, *in situ* crystallization, and the like. Suitable catalytic materials for use herein include metals, in particular metals that are capable of forming a carbide or that are capable of dissolving carbon. Preferred are transition metals, in particular (for cost reasons) non-noble transition metals. Most preferred for use herein are metals from the group consisting of Fe, Co, Ni, Cr, V, Mo, and mixtures thereof.

The carbon source may be a gas or a liquid. Examples of gaseous carbon sources include methane, carbon monoxide, synthesis gas (a mixture of carbon monoxide and hydrogen), ethyne, ethane, and mixtures thereof. The subsequent conversion of biomass produces liquid and/or gaseous products which, for cost reasons, are particularly preferred for use as the carbon source in the present process.

Although the inventor does not wish to be bound by theory, it is believed that carbon from the carbon source forms a carbide with the catalytic material, and/or dissolves in the catalytic material. The carbon migrates through the catalytic material and forms carbon fibers at one of the surfaces of the catalytic particle, for example the surface that is in contact with the biomass particle. The carbon fiber may grow along the surface of the biomass particle, or even penetrate into the biomass particle to become embedded within the particle. The process is carried out at temperatures in the range of 200 to 1100 °C, preferably from 300 to 600°C.

The term "associated with carbon fibers" or "associated with fibers of carbon" as used herein refers to biomass particles that have carbon fibers coated onto their surface and/or embedded within the particles. The term covers such particles made by the process disclosed herein, or by some other process. It will be understood that the term "coated" does not require that the surface of the particles is fully covered with carbon fibers; it merely connotes a situation of biomass particles having carbon fibers attached to their surface. The carbon fibers may be attached to the biomass particles at one single point, or at several points, or along their entire length. The term "fibers" also encompasses fiber-like structures, such as tubes.

Biomass particles associated with carbon fibers possess an increased susceptibility to conversion to, for example, a bioliquid as compared to unmodified biomass particles of similar composition and size. Without wishing to be bound by theory, the inventor believes that there are several possible reasons for this increased susceptibility. Firstly, the carbon fibers present catalytically active sites that may be involved in subsequent conversion reactions. Secondly, the carbon fibers penetrate the surface of the biomass particle at the point or points where the fiber is attached to the surface, thereby making the biomass more accessible to chemical reactions. This effect is even more pronounced for particles that have carbon fibers embedded therein.

Accordingly, another aspect of the present invention is the use of biomass particles associated with carbon fibers in processes for preparing a bioliquid therefrom. Examples of suitable processes include hydrothermal conversion, pyrolysis, enzymatic conversion, catalytic conversion, and mild thermal conversion. Mild thermal conversion may be carried out in the presence of hydrogen.

Specifically, one aspect of the present invention is a process for preparing a bioliquid from a solid biomass material, said process comprising the steps of:
a) providing the solid biomass in a particulate form;
b) activating said particulate biomass by associating carbon fibers therewith;
c) subjecting said activated biomass to hydrothermal conversion at a temperature below 300 °C.

Preferably step c) is carried out at a temperature below 240 °C, more preferably below 200 °C.

Another aspect of the present invention is a process for preparing a bioliquid from a solid biomass material, said process comprising the steps of:
a) providing the solid biomass in a particulate form;
b) activating said particulate biomass by associating carbon fibers with said biomass;
c) subjecting said activated biomass to an enzymatic conversion

Preferably, in step b) the carbon fibers are embedded within the solid biomass.

Another aspect of the present invention is a process for preparing a bioliquid from a solid biomass material, said process comprising the steps of:
a) providing the solid biomass in a particulate form;
b) activating said particulate biomass by associating carbon fibers with said biomass;
c) subjecting said activated biomass to a mild thermal conversion.

Preferably, in step b) the carbon fibers are embedded within the solid biomass.

Preferably step c) is performed in the presence of hydrogen.

## Claims

1. A particulate solid biomass material having associated therewith fibers of carbon.

2. The particulate solid biomass material of claim 1 wherein the fibers of carbon are coated onto the particulate biomass material.

3. The particulate solid biomass material of claim 1 wherein the fibers of carbon are embedded within the particulate biomass material.

4. The biomass material of any one of the preceding claims wherein the fibers are carbon nanofibers.

5. The biomass material of any one of the preceding claims which is of photosynthetic origin.

6. The biomass material of claim 5 which comprises cellulose and/or lignocellulose.

7. The biomass material of any one of the preceding claims which is susceptible to hydrothermal conversion at a temperature below 300 °C.

8. The biomass material of claim 7 which is susceptible to hydrothermal conversion at a temperature below 240°C.

9. The biomass material of claim 7 which is susceptible to hydrothermal conversion at a temperature below 200 °C.

10. A process for preparing a solid biomass material having associated therewith fibers of carbon, said process comprising the steps of:
a) providing a solid biomass material in particulate form;
b) depositing onto said particulate biomass material particles of a catalytic material capable of catalyzing the formation of carbon fibers, to form an activated biomass;
c) contacting the activated biomass with a suitable carbon source.

11. The process of claim 10 wherein the carbon fibers are coated onto the solid biomass particles.

12. The process of claim 10 wherein the carbon fibers are embedded within the biomass particles.

13. The process of any one of claims 10-12 wherein the catalytic material comprises a metal capable of forming a carbide or capable of dissolving carbon.

14. The process of claim 13 wherein the metal is a transition metal.

15. The process of claim 14 wherein the metal is a non-noble transition metal.

16. The process of claim 15 wherein the metal is selected from the group consisting of Fe, Co, Ni, Cr, V, Mo, and mixtures thereof.

17. The process of any one of claims 10-16 wherein the carbon source comprises a gaseous carbon source.

18. The process of claim 17 wherein the carbon source comprises a gas selected from the group consisting of methane, carbon monoxide, synthesis gas, ethyne, ethane, and mixtures thereof.

19. The process of any one of claims 10-16 wherein the carbon source comprises a liquid.

20. The process of any one of claims 10-19 wherein the carbon source comprises a decomposition product of the solid biomass.

21. The process of claim 20 wherein the decomposition product of the solid biomass is a gas.

22. The process of claim 21 wherein the decomposition product af the solid biomass is a liquid.

23. A process for preparing a bioliquid from a solid biomass material, said process comprising the steps of:
a) providing the solid biomass in a particulate form;
b) activating said particulate biomass by associating carbon fibers with said biomass;
c) subjecting said activated biomass to hydrothermal conversion at a temperature below 300 °C.

24. The process of claim 23 wherein the temperature in step c) is less than 240 °C.

25. The process of claim 24 wherein the temperature in step c) is less than 200°C.

26. A process for preparing a bioliquid from a solid biomass material, said process comprising the steps of:
a) providing the solid biomass in a particulate form;
b) activating said particulate biomass by associating carbon fibers with said biomass;
c) subjecting said activated biomass to an enzymatic conversion

27. The process of claim 26 wherein the carbon fibers are embedded within the solid biomass.

28. A process for preparing a bioliquid from a solid biomass material, said process comprising the steps of:
a) providing the solid biomass in a particulate form;
b) activating said particulate biomass by associating carbon fibers with said biomass;
c) subjecting said activated biomass to a mild thermal conversion.

29. The process of claim 25 wherein the carbon fibers are embedded within the solid biomass.

30. The process of claim 28 or 29 wherein the conversion step c) is performed in the presence of hydrogen.
